Europäisches Patentamt

European Patent Office

Office européen des brevets

(11) Veröffentlichungsnummer : **0 092 798**
**B1**

(12) **EUROPÄISCHE PATENTSCHRIFT**

(45) Veröffentlichungstag der Patentschrift :
**01.10.86**

(21) Anmeldenummer : **83103881.5**

(22) Anmeldetag : **20.04.83**

(51) Int. Cl.⁴ : **A 61 N   1/05, A 61 N   1/362,**
**H 01 B 11/18, H 01 B   7/04**

(54) **Mehrpolige koaxiale Leitung.**

(30) Priorität : **22.04.82 DE 3215036**

(43) Veröffentlichungstag der Anmeldung :
**02.11.83 Patentblatt 83/44**

(45) Bekanntmachung des Hinweises auf die Patenterteilung : **01.10.86 Patentblatt 86/40**

(84) Benannte Vertragsstaaten :
**DE FR GB IT NL SE**

(56) Entgegenhaltungen :
**EP-A- 0 022 556**
**CH-A-   547 553**
**DE-A- 2 116 364**
**DE-A- 2 408 707**
**DE-A- 2 820 867**
**DE-A- 2 915 740**
**DE-A- 3 031 752**
**DE-A- 3 046 732**
**DE-B- 1 078 653**
**DE-C-   906 587**
**FR-A-   928 287**
**GB-A- 1 256 640**
**US-A- 3 601 721**

(73) Patentinhaber : **Siemens-Elema AB**
**Röntgenvägen 2**
**S-171 95 Solna 1 (SE)**
**SE**
**Siemens Aktiengesellschaft Berlin und München**
**Wittelsbacherplatz 2**
**D-8000 München 2 (DE)**
**DE FR GB IT NL**

(72) Erfinder : **Hirschberg, Jakub**
**Aprilvägen 3**
**S-183 44 Täby (SE)**

## Beschreibung

Die Erfindung betrifft eine mehrpolige koaxiale Elektrodenleitung gemäss dem Oberbegriff des Patentanspruches 1.

Bekannte koaxiale Leitungen dieser Art bestehen aus zwei durch einen Isolierschlauch voneinander getrennten spiralförmig gewickelten Drähten, die aussen ebenfalls von einem Isolierschlauch umgeben sind. Eine derartige Elektrodenleitung ist aus der Druckschrift DE-A-2 820 867 etsichtlich. Da heutzutage vorhandene Stimulatoren, beispielsweise Herzschrittmacher, eine sehr hohe Lebensdauer haben, werden insbesondere bei implantierten Systemen hohe Anforderungen an die Elektrodenleitungen gestellt. Die Leitungen sollen geschmeidig sein, um möglichst geringe Organstörungen hervorzurufen. Weiterhin soll der Widerstand klein sein, um den Energieverbrauch niedrig zu halten und damit die Lebensdauer der Batterien zu erhöhen. Die Leitungen müssen eine hohe Biegedauerfestigkeit aufweisen.

Die mechanischen Eigenschaften der Leitung werden entscheidend durch den Durchmesser der Leitung bestimmt. Dieser liesse sich im Prinzip durch Verringerung der Drahtquerschnitte vermindern. Das würde aber auf der anderen Seite zu einem unerwünscht hohen Widerstand führen. Aus demselben Grund muss bei den bekannten koaxialen Leitungen bereits der Durchmesser des äusseren Drahtes grösser sein als der des inneren, da jener länger ist. Ein üblicher Wert für den Durchmesser einer koaxialen Leitung mit Silikongummi als äussere Isolierung ist 2,8 mm.

Um den Durchmesser weiter zu vermindern, ist aus der DE-A-3 031 752 bekannt, die Leiter einzeln zu isolieren und multifilar zu einer Wendel zu wickeln. Dadurch ist es zwar gelungen, den Druchmesser der Leitung herabzusetzen, gleichzeitig bekommt man aber eine flachere Steigung der Wendel und damit eine verminderte Biegsamkeit.

Zur Verringerung des Durchmessers ist weiterhin aus der EP-0 022 556-A 1 eine koaxiale Elektrodenleitung bekannt, die bandförmige Leiterwendeln aufweist.

Der vorliegenden Erfindung liegt die Aufgabe zugrunde, eine im Aufbau einfache, mehrpolige koaxiale Elektrodenleitung anzugeben, bei der unter Beibehaltung hoher Flexibilität und Dauerbiegefestigkeit sowie geringem Totaldurchmesser, die sichere Einführung eines Führungsdrahtes ohne zusätzliche Massnahmen möglich ist.

Diese Aufgabe wird erfindungsgemäss dadurch gelöst, dass die auf die innerste Drahtwendel aufgebrachte(n) Leiterwendel(n) aus Metallband besteht (bestehen). Diese erfindungsgemässe Elektrodenleitung stellt eine geschickte Kombination einer bekannten drahtförmigen Leiterwendel als Innenleiter dar, auf die getrennt über eine Isolierschicht mindestens eine zweite Leiterwendel aus Metallband aufgebracht ist. Die innere Leiterwendel dient damit gleichzeitig als Träger oder Stütze für das dünne Metallband. Man erreicht damit eine mindestens zweipolige koaxiale Elektrodenleitung mit verringertem Durchmesser, mindestens ebensogute elektrische Leitfähigkeit für den äusseren Leiter trotz des geringeren Durchmessers und eine verbesserte Flexibilität bei grössere Dauerbiegefestigkeit gegenüber Koaxialleitungen ausschliesslich aus Drahtwendeln. Durch Beibehalten des drahtförmigen Leiters für die innere Leiterwendel erhält man ohne zusätzliche Hilfsmassnahmen eine einwandfreie Öffnung für den Führungsdraht, durch die sich dieser einfach und ungehindert einführen lässt. Der Durchmesser des gesamten Leiters lässt sich bei ansonsten unverändertem Aufbau bereits auf 2,2 mm herabsetzen.

Unter Metallband soll in diesem Zusammenhang auch eine bandförmige Litze aus geflochtenen Drähten verstanden werden.

Um den Widerstand bei den Leitern gemäss der vorliegenden Erfindung noch weiter zu verringern und die Biegsamkeit zu verbessern, ist in Weiterbildung der Erfindung vorgesehen, dass mindestens ein Leiter multifilar aufgebaut ist.

Vorteilhafterweise sollen die Kanten der verwendeten Metallbänder abgerundet sein. Dadurch werden bereits bei der Herstellung und später auch während des Betriebes der Leitung Beschädigungen der Isolierschichten vermieden, die die Lebensdauer der Leitung herabsetzen könnten.

Eine gute Stabilität der Leitung ergibt sich bei bleibend hoher Biegsamkeit auch dann, wenn die Isolierschicht als Träger für das Metallband beziehungsweise die Metallbänder dient. Dabei kann auf beiden Seiten der Isolierschicht ein Metallband oder mehrere aufgebracht sein. Die Isolierschicht kann gleichfalls ein Band sein. Für eine vereinfachte Herstellung kann ein Isolierband mit einer geforderten Anzahl Metallbänder als Ausgangsmaterial gewählt werden, das dann nur noch wendelförmig um den inneren Drahtleiter zu werden braucht.

An Hand einer Figur wird im folgenden ein Ausführungsbeispiel näher beschrieben und erläutert. Die einzige Figur zeigt dabei eine bipolare Leitung mit einer inneren Drahtwendel und einem äusseren bandförmigen Leiter.

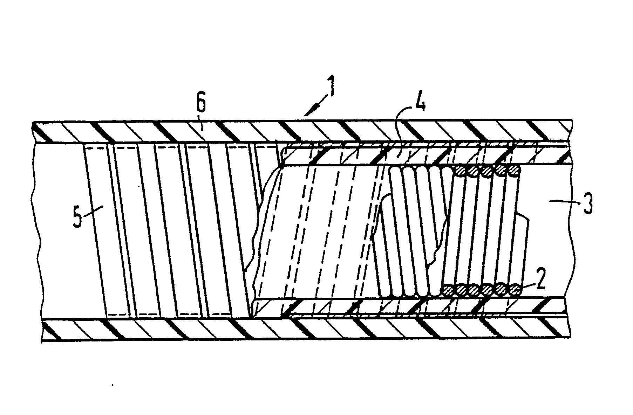

In Figur 1 ist teilweise im Schnitt eine bipolare Leitung 1 dargestellt, die im Inneren einem spiralförmigen Leiter 2 aus möglichst leitfähigem und biegedauerbeständigem Draht aufweist. Dieser Leiter 2 bildet mit seinem Inneren eine zentrale Öffnung 3 für einen nicht dargestellten Führungsdraht (Mandrin). Aussen ist der Leiter 2 von einem ersten Isolierschlauch 4 umgeben. Auf diesem Isolierschlauch befindet sich als zweiter Leiter ein wendelförmig gewickeltes Metallband 5, dessen Dicke wesentlich kleiner als der Durchmesser des Drahtes für den Leiter 2 ist, dessen Widerstand aber trotzdem dem des Leiters 2 entspricht. Den äusseren Abschluss bildet ein weiterer Isolierschlauch 6.

Die Kanten des bandförmigen Leiters können abgerundet sein. Als mögliches Material für die Leiter mit geringem Widerstand und hoher Biegedauerfestigkeit können z. B. extrudiertes Kupfer mit Tantalbeschichtung oder verschiedene Legierungen von Nickel, Kobalt, Chrom, Molybdän und u. U. Eisen verwendet werden. Für die inneren Isolierschichten kann beispielsweise Silikongummi, Polyäthylen, thermoplastisches Polyurethan oder thermoplastisches Polytetrafluoräthylen verwendet werden. Für die äusserste, mit Körpergewebe oder Körperflüssigkeit in Berührung kommende Isolierschicht eignet sich vorteilhaft Silikongummi oder thermoplastisches Polyurethan.

Neben dem dargestellten Ausführungsbeispiel sind im Rahmen der Erfindung auch Variationen davon denkbar. So kann die Zahl der Leiter bei Bedarf weiter gesteigert werden, wobei sich der Vorteil geringerer Durchmessersteigerung der Koaxialleitung immer stärker auswirkt.

**Patentansprüche**

1. Mehrpolige koaxiale Elektrodenleitung (1) für einen implantierbaren Herzschrittmacher mit durch jeweils mindestens eine Isolierschicht (4) voneinander getrennten, koaxial verlaufenden elektrischen Leiterwendeln (2, 5) sowie mit einer zentralen Öffnung (3) für einen Führungsdraht, wobei die innerste Leiterwendel (2) aus Draht besteht dadurch gekennzeichnet, dass die auf diese innerste Drahtwendel aufgebrachte(n) Leiterwendel(n) (5) aus Metallband besteht (bestehen).

2. Mehrpolige Elektrodenleitung nach Anspruch 1, dadurch gekennzeichnet, dass mindestens ein Leiter multifilar aufgebaut ist.

3. Mehrpolige Elektrodenleitung nach einem der Ansprüche 1 oder 2, dadurch gekennzeichnet, dass die Isolierschicht bandförmig ausgebildet ist und als Träger für mindestens ein Metallband dient.

4. Mehrpolige Elektrodenleitung nach Anspruch 3, dadurch gekennzeichnet, dass das Metallband auf beiden Seiten des Trägers aufgebracht ist.

**Claims**

1. A multi-polar coaxial electrode lead (1) for an implantable heart pacemaker, comprising coaxially arranged, electrical conductor coils (2, 5) which are each separated from one another by at least one insulating layer (4), and comprising a central opening (3) for a guide wire, wherein the innermost conductor coil (2) comprises wire, characterised in that the conductor coil(s) (5) applied to this innermost wire coil comprises(comprise) strip metal.

2. A multi-polar electrode lead as claimed in claim 1, characterised in that at least one conductor is of multifilament construction.

3. A multi-polar electrode lead as claimed in one of claims 1 or 2, characterised in that the insulating layer is of strip formation and serves as a carrier for at least one metal strip.

4. A multi-polar electrode lead as claimed in claim 3, characterised in that the metal strip is applied to both sides of the carrier.

**Revendications**

1. Conducteur coaxial multipolaire (1) pour électrode pour un stimulateur cardiaque implantable, comportant des conducteurs électriques hélicoïdaux (2, 5) disposés coaxialement et séparés les uns des autres par respectivement au moins une couche isolante (4), ainsi qu'une ouverture centrale (3) pour un fil de guidage, le conducteur hélicoïdal (2) le plus intérieur étant constitué par un fil, caractérisée par le fait que le ou les conducteurs hélicoïdaux (5) disposés sur ce fil hélicoïdal le plus intérieur sont constitués par une bande métallique.

2. Ligne multipolaire pour électrode suivant la revendication 1, caractérisée par le fait qu'au moins un conducteur est constitué par plusieurs fils.

3. Ligne multipolaire pour électrode suivant l'une des revendications 1 ou 2, caractérisée par le fait que la couche isolante est réalisée en forme de bande et sert de support pour au moins une bande métallique.

4. Ligne multipolaire pour électrode suivant la revendication 3, caractérisée par le fait que la bande métallique est disposée sur les deux faces du support.